# EUROPEAN PATENT SPECIFICATION

(11) **EP 1 867 673 B1**
(45) Date of publication and mention of the grant of the patent: **16.11.2011**
(21) Application number: 06729717.6
(22) Date of filing: 16.03.2006
(51) Int. Cl.: C08G 65/333, A61K 31/282, A61P 35/00, A61K 47/48

(54) **COORDINATION COMPOUND COMPOSED OF DIAMINOCYCLOHEXANE PLATINUM (II) AND BLOCK COPOLYMER AND ANTI-CANCER AGENT COMPRISING THE SAME**
KOORDINATIONSVERBINDUNG AUS DIAMINOCYCLOHEXAN-PLATIN(II) UND BLOCKCOPOLYMER UND DIESE ENTHALTENDES ANTIKREBSMITTEL
COMPOSE DE COORDINATION COMPOSE DU DIAMINOCYCLOHEXANE PLATINE (II) ET D'UN COPOLYMERE SEQUENCE ET AGENT ANTICANCEREUX LE COMPRENANT

(30) Priority: 18.03.2005 JP 2005080117
(43) Date of publication of application: 19.12.2007
(73) Proprietor: The University of Tokyo, Bunkyo-ku, Tokyo 113-8654 (JP)
(72) Inventor: KATAOKA, Kazunori, Tokyo, 1138654 (JP); NISHIYAMA, Nobuhiro, Tokyo, 1138654 (JP); CABRAL, Horacio, Tokyo, 1138654 (JP)
(74) Representative: Albrecht, Thomas
(86) International application number: PCT/JP2006/305750
(87) International publication number: WO 2006/098496

(56) References cited:
- WO-A1-02/26241
- WO-A1-2005/056641
- JP-A- 05 301 884
- JP-A- 06 271 593
- JP-A- 06 329 692
- JP-A- 11 100 331
- JP-A- 57 002 296
- FURIN A. ET AL.: 'Synthesis, characterization and preliminary cytotoxicity assays of poly(ethylene glycol)-malonato-Pt-DACH conjugates' EUROPEAN JOURNAL OF MEDICINAL CHEMISTRY vol. 38, 2003, pages 739 - 749, XP004448325

## Description

### Technical Field

This invention relates to a coordination compound comprising at least one (1,2-diaminocyclohexane)platinum (II) moiety and at least one block copolymer moiety derived from poly(ethylene glycol)-poly(glutamic acid) block copolymer [hereafter may be abbreviated as PEG-P(Glu)]; to anti-tumor agents or medical preparations for treating cancer, which contain the coordination compound as the active ingredient; and furthermore to a method for treating cancer patients.

### Background Art

Recent progress in drug delivery system (DDS) as a system for having a required amount of a drug act at the required site and time is gathering keen attention in the clinical art, as being a methodology for accomplishing safe and effective drug therapy. Research on DDS is directed mainly to controlling drug supply to intended target sites in living bodies, or controlling drug distribution in vivo. In the latter, ribosomes, microspheres and various water-soluble polymers have been investigated as drug carriers. For a DDS to be successfully used, it must be capable of protecting the drug until it reaches the target site, from non-specific elimination due to, for example, uptake by the reticuloendothial system (RES) and, at the same time, capable of rapid drug transfer in blood circulation to the affected tissues.

A part of the present inventors have developed a DDS in which a nanometer size polymolecular association product (polymeric micelles) of a specific hydrophilic-hydrophobic block copolymer is loaded with, or encapsulating, a drug, in particular, an anti-cancer agent (adriamycin, cisplatin or the like), as a system wherein the carrier itself is less recognizable by RES and exhibits high blood vessel permeability. Utilization of the system has enabled successful accumulation of the drug in the targetted solid cancer cells or tissues with high efficiency (cf. Patent document 1 or 2 for adriamycin; Non-patent document 1 or Patent document 3 or 4 for cisplatin. These documents and later-cited documents are collectively listed later).

Cisplatin (cis-diamminedichloroplatinum; CDDP) has been widely used as an anti-cancer drug, regardless of its potent toxicity, because it is effective particularly for treating cancer of genital organs. The CDDP-encapsulating polymer micelles which are described in Patent document 3 or 4 are considered to be very promising anti-cancer agents in that they have markedly reduced toxicity in comparison with free CDDP and furthermore can semi-specifically accumulate in cancer cells or tissues. Besides the CDDP-encapsulating polymeric micelles, various CDDP derivatives or analogues have been proposed to date, since 1960's when CDDP was discovered. Of those derivatives or analogues, particularly dichloro(1,2-diaminocyclohexane)platinum (II) (DACHPt) has gathered attention because of its wider and unique spectrum of activity than CDDP, such as lower toxicity than CDDP and no cross resistance with CDDP in many CDDP-resistant cancers.

Whereas, DACHPt has not yet been put to clinical use, one of the reasons therefor being its very low solubility in water (cf. Non-patent document 2). With the view to improve water solubility of DACHPt, a large variety of derivatives or analogues were proposed, among which oxalate (1,2-diaminocyclohexane)platinum (II) (which is also called oxaliplatin) has been clinically used in considerable amounts in various countries of the world, after having been evaluated by clinical trials (cf. e.g., Non-patent document 3) and approved of its manufacture and marketing by the Food and Drug Administration in U.S.A. in 2002 as a third generation platinum drug. This is due to the fact that oxaliplatin is nearly free from those problems in stability, water solubility, formulation and safety which are found with other various DACHPt derivatives, or is subject to such problems by drastically less seriousness.

Whereas, if a DACHPt derivative having further improved characteristics as an anti-cancer drug could be provided, it will contribute to advance chemotherapy of cancer. Comparatively recently, poly(ethylene glycol)-malonate-Pt-DACH conjugate was proposed (cf., for example, Non-patent document 4). This Non-patent document 4 discloses that the conjugate succeeded in markedly increasing water solubility of DACHPt, and its better anti-tumor activity than DACHPt is suggested by a therapy protocol of intraperitoneal administration of drugs to tumor-implanted laboratory animals. The document also shows that the authors' platinum release tests in phosphate buffer solution (PBS) at pH 7.4 confirmed more than 50% Pt-DACH release within 2 hours. It will be difficult to expect the conjugate of Non-patent document 4, which shows such release behavior, to have a long retention time in blood circulation when it is, for example, intravenously administered. Furthermore, it is not necessarily certain that the conjugate can provide a better anti-cancer drug than oxaliplatin, although it is yet indefinite because the result of the conjugate's clinical trial is not compared with that of oxaliplatin which is held to have the best characteristics among the analogues.

Once again, characteristics of anti-cancer drug which we consider favorable are, as aforesaid, first, the DDS or drug derivative is capable of protecting the drug from non-specific uptake by RES or the like, whereby enabling its stable and prolonged presence in blood circulation; and second, for the DDS or drug derivative to actually exhibit therapeutic effect, it must have high stability in biological media such as blood, but once it is delivered to the target site, it can effectively release the active drug at that site. This first special property and the second special property may be regarded incompatible. Surprisingly, however, Patent documents 1 and 3 could provide conjugates of drug and block copolymer, which concurrently possess both of the properties to an extent allowing their clinical use.

The conjugate relevant to DACHPt as described in Non-patent document 4, in which the two chloro ligands of DACHPt are substituted with two carboxylate groups of the malonate bound to poly(ethylene glycol) terminal, shows premature release of Pt-DACH in PBS, and hence is unreliable as to stability in biological media, one aspect of the above-described second specific property.

Thus, although the attempt in Patent document 3 was successful with CDDP, there is no guaranteeing that the use of poly(ethylene glycol)-block-poly(glutamic acid) (PEG-P(Glu)) block copolymer which also utilizes formation of coordination bond through PEG-modified polycarboxylic acid, would bring about favorable result with DACHPt.

Whereas, when two chloro ligands of DACHPt are substituted with neutral aqua ligands in advance and a compound falling within a concept of coordination compound or coordination complex is formed in water, using PEG-P(Glu) (Mw of PEG segment = 12,000; degree of polymerization of P(Glu) = 46; and molecular weight distribution, Mm/Mn = 1.06), normally polymeric micelles of the compound are formed accompanying the production of such coordination compound or coordination complex. Surprisingly, we have confirmed that the so obtained polymeric micelle (conjugate- or DACHPt-encapsulating polymeric micelle) initiates sustained platinum complex release only after 12 hours' induction period in phosphate buffered physiological saline at 37°C (cf. Non-patent document 5). Non-patent document 5 also discloses: surprisingly, in spite of the extremely high stability in physiological media as above, the polymeric micelle complex achieved markedly higher platinum accumulation in tumor than oxaliplatin in the intravenous administration tests with colon cancer C-26 cell line-bearing CDF₁ mice; and its in vitro cytotoxicity to colon cancer C-26 cell line was much higher than that of oxaliplatin.

### List of cited documents

- Patent document 1:: Japanese Patent No. 2517760
- Patent document 2:: EP 0397307 A1
- Patent document 3:: WO 02/026241
- Patent document 4:: EP 1329221 A1
- Non-patent document 1:: N. Nishiyama, et al., Langmuir 15 (1999) 377-383
- Non-patent document 2:: Y. Kidani, et al., J. Med. Chem. 21 (1978) 1315-1318
- Non-patent document 3:: E. Raymond, et al., Molecular Cancer Chemotherapy 1 (2002) 227-235
- Non-patent document 4:: A. Furin, et al., Eur. J. Med. Chem. 11 (2003) 5443-5447
- Non-patent document 5:: H. Cabral, et al., Journal of Controlled Release, 101 (2005) 223 - 232

EP-A-1 695 991 discloses coordination compounds comprising a (1,2-diaminocyclohexane)-platinum (II) moiety and a moiety derived from a block copolymer [PEG·P(Glu)] (cf. Example 1 wherein PEG stands for poly(ethylene glycol) and P(Glu) for poly(glutamic acid)). The compound of Example 1 of EP-A-1 695 991 has a number of repeating units of P(Glu) equal to 35.

### Disclosure of the Invention

The DACHPt-loaded or -encapsulating polymeric micelles that are disclosed in Non-patent document 5 are promising candidate of anti-cancer agent having the characteristics surpassing even oxaliplatin. Should there be provided, however, an anti-cancer drug which can be used more safely than any of conventional platinum-derived anti-cancer agents including the one disclosed in Non-patent document 5, and which moreover exhibits still higher efficacy, it will contribute to further progress in chemotherapy of cancer.

The DACHPt-encapsulating or -loaded polymeric micelles as described in Non-patent document 5 have better characteristics than oxaliplatin as anti cancer drug, but the needs for obtaining compounds which exhibit even more enhanced efficacy still exist.

Under the circumstances, we have engaged in further investigations of relevancy between structure and medical efficacy of DACHPt-encapsulating or DACHPt-loaded polymeric micelles, and in the course of the research, prepared for trial DACHPt conjugates with PEG-P(Glu) copolymers of significantly shorter chain length than the PEG-P(Glu) copolymer as described in Non-patent document 5 in which the degree of polymerization of P(Glu) was 46, the copolymers having PEG chains of a length sufficient for forming polymeric micelles in aqueous media, although it was generally thought that the shorter the P(Glu) chains to carry the carboxyl groups which are the ligands to platinum, the less would be the stability of the resulting coordination compound when the formation mechanism of a coordination compound was considered. In consequence, we could confirm: while the variation in the PEG chain length little affects stability of the resulting conjugate (coordination compound) or the polymeric micelle derived from the coordination compound in aqueous media, DACHPt conjugates formed with the copolymers in which the average degree of polymerization of P(Glu) chains or average number of Glu repeating units is not more than 30; but at least 15, preferably at least 17, more preferably, at least 20, surprisingly could not only retain the required stability in the physiological medium but also significantly reduce the toxicity associated with the drug's accumulation in healthy organs including the liver, and still in addition, exhibit high anti-cancer effect in vivo.

Accordingly, therefore, the present invention provides a coordination compound comprising (1,2-diaminocyclohexane)-platinum (II) moiety and block copolymer moiety derived from a block copolymer of the general formula (1-a) or (1-b): 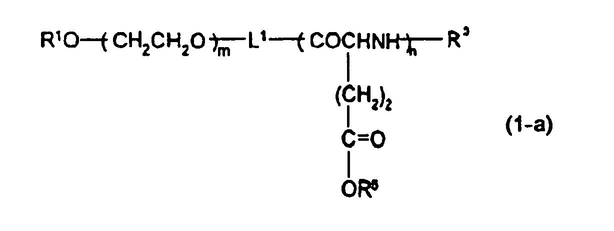 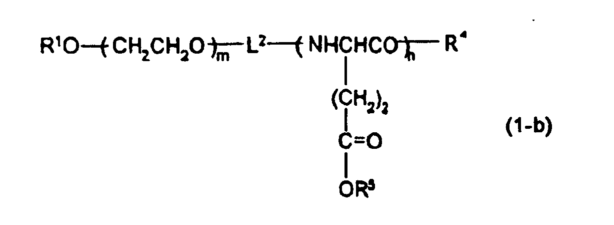 (in the formulae, R¹ stands for hydrogen atom or an unsubstituted or substituted, straight chain or branched chain C₁₋₁₂ alkyl; L¹ and L² each stands for a linking group; R³ stands for hydrogen atom, protecting group of amino group, hydrophobic group or polymerizable group; R⁴ stands for hydroxyl or an initiator residue; R⁵ each independently stands for hydrogen atom, an alkali metal ion or protecting group of carboxyl group; m is an integer of 35 - 20,000; and n is an integer in the range of 15 to 30, preferably 17 - 30, with the proviso that hydrogen or an alkali metal ion occupies at least 80% of n R⁵s)
(that is, the hydrogen atoms or alkali metal ions occupying at least 80% of R⁵s in the block copolymer are partially or entirely removed), the coordination compound being formed through a coordination bond between the platinum in the (1,2-diaminocyclohexane)platinum(II) moiety and one or two carboxylate groups in the block copolymer moiety.

As preferred embodiments of the present invention, the coordination compounds in which, referring to the given general formula (1-a) or (1-b),
(i) all of R⁵s stand for hydrogen atom or an alkali metal ion;
(ii) where the coordination bond is formed through the platinum in the (1,2-diaminocyclohexane)platinum(II) moiety and one carboxylate group in the block copolymer moiety, aqua or chloro ligand(s) bind to the remaining bonding hand(s) of the platinum;
(iii) L¹ is -(CH₂)_{b}-NH-, b being an integer of 1 - 5 and L² is - (CH₂)_{c}-CO-, c being an integer of 1 - 5;
(iv) m is an integer of 40 - 500, and n is 17 - 25;
(v) the coordination compound is capable of forming, in an aqueous medium, a polymeric micelle in such a form that nanoparticles composed of the poly(ethylene glycol) segments in the block copolymer of the general formula (1-a) or (1-b) serving as the shell and the poly(glutamic acid) segments serving as the core, encapsulate 1,2-diaminocyclohexane platinum(II); or
(vi) the equivalent ratio (Pt/COO-) of platinum(Pt) in the (1,2-diaminocyclohexane)platinum (II) moiety to carboxyl group in the block copolymer moiety is 0.3 - 1; are provided.

As another embodiment, the present invention provides anti-cancer compositions comprising above-described coordination compound and pharmaceutically acceptable excipients.

As still another embodiment, the present invention provides use of above-described coordination compound for formulating medical preparations for treating cancer patients.

The present invention furthermore provides a coordination compound as defined above for use in a method for treating cancer patients.

### Detailed Description of the Invention

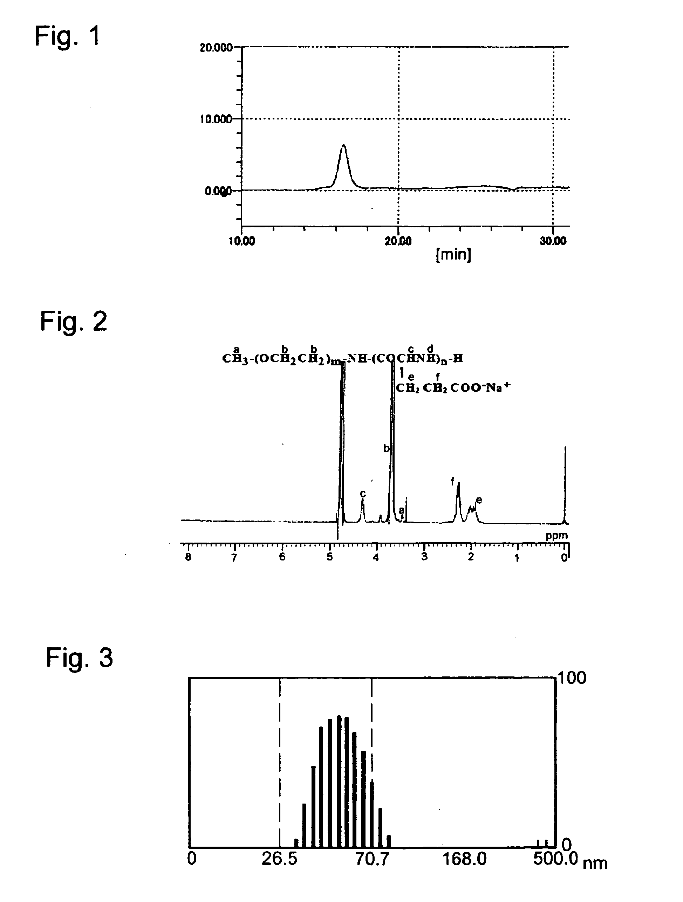

In the coordination compound (which may hereafter be referred to as coordination complex) according to the present invention, at least one of the two chloros in a leaving group (or ligand) from DACHPt is substituted with a side chain carboxylate of P(Glu) segment, and one of the two chloros may be aquotized (meaning the state that a water molecule is coordinated with platinum ion by means of the lone pair in its oxygen atom) or remain to be chloro. In the complex, the other ligand of DACHPt, 1,2-diaminocyclohexane, remains as it is. Where the two chloros are substituted with two carboxylate groups, the two carboxylate groups can be derived from an adjacent or considerably remote single polymer molecule, or from plural polymer molecules. Although not limited thereto, Fig. 2 of Non-patent document 5 can be referred to concerning the structural formula of the coordination compounds and schematic illustration of the DACHPt-encapsulating micellar structure which the compounds are presumed to form in an aqueous medium.

The DACHPt moiety can be any of various stereoisomers as stated in Non-patent document 2, and so long as the object of the present invention is met, it can be any one of such isomers or a mixture of the isomers. It is preferable, however, that the configuration of the moiety follows that of oxaliplatin.

Definitions of individual groups or moieties in the general formulae must be construed in line with the significations or contents normally accepted by skilled artisans. Although not to be taken as limitative, specific examples are given hereinbelow.

Examples of R¹ which stands for unsubstituted or substituted, straight chain or branched chain C₁₋₁₂ alkyl include, methyl, ethyl, n-propyl, iso-propyl, n-butyl, sec-butyl, tert-butyl, n-pentyl, n-hexyl, decyl, undecyl and the like. Where they are substituted, the substituent includes, acetalized formyl group, cyano group, formyl group, carboxyl group, amino group, C₁₋₆ alkoxycarbonyl group, C₂₋₇ acylamido group, the same or different tri-C₁₋₆ alkylsiloxy group, siloxy group or silylamino group. When the substituent is acetalized formyl, it can be hydrolyzed under mild acidic conditions to be converted to another substituent group, formyl (-CHO: or aldehyde group). The formyl, or carboxyl or amino as named among above examples can be utilized to impart target directivity to the coordination compound of the present invention through the steps, for example, of introducing such a group into the formed coordination compound by deprotection or conversion of corresponding protected group or moiety and, where necessary, covalently bonding therewith a suitable antibody or a fragment thereof having its specific binding property (F(ab)₂, F(ab), or folic acid and the like). A PEG segment having such a functional group at one of its terminals can be conveniently formed by the method of producing PEG segments of block copolymers as described in, for example, WO 96/32434, WO 96/33233 or WO 97/06202.

Linking of thus formed PEG segments with P(Glu) segments can be effected by any suitable mode, according to the method used for producing the block copolymer of the general formula (1-a) or (a-b) and, so long as the object of the present invention can be accomplished, any linking group can be used for the linkage. While the production method is not limited, as a method for preparing a complex of the general formula (1-a) or (1-b), a process using a PEG derivative having a terminal amino group can be utilized, the process comprising synthesizing a block copolymer by ring-opening polymerization of the PEG derivative with N-carboxylic acid anhydride (NCA) of γ-benzyl-L-glutamate, initiated at the amino terminal, and thereafter obtaining the object block copolymer by converting the side chain benzyl groups to other ester groups, or partially or completely hydrolyzing them. In this case the copolymer comes to have a structure represented by the general formula (1-a) and the linking group L¹, a structure derived from the terminal structure of the PEG segment used, which preferably is -(CH₂)_{b}-NH-(wherein b is an integer of 1 - 5).

The copolymer of the present invention can also be produced by a method of synthesizing P(Glu) segments and then linking them with previously prepared PEG segments, in which case the copolymer may eventually take a same structure with that of the product of previously described method, or a structure corresponding to the general formula (1-b). The kind of the bonding group L² is not limited, but preferably is -(CH₂) or -CO- (wherein c is an integer of 1 -5).

R⁵s in the general formula (1-a) or (1-b) can be each independently hydrogen atom or a protecting group of carboxyl group. As examples of the protecting group of carboxyl group, although not limited thereto, benzyl, benzhydrile and C₁₋₆ alkyl group can be named, specific examples of the alkyl group including methyl, ethyl, n-propyl, iso-propyl, n-butyl, sec-butyl, tert-butyl, n-pentyl and n-hexyl.

R³ can be each independently hydrogen atom, a protecting group of amino group such as benzyloxycarbonyl, t-butyloxycarbonyl, acetyl or trifluoroacetyl; a hydrophobic group such as benzylcarbonyl or benzhydrylcarbonyl; or a polymerizable group such as acryloyl or methacryloyl.

R⁴ can be each independently a hydroxyl- or carboxyl-protective group, such as benzyloxy, t-butyloxy or methoxy; a hydrophobic group such as benzyloxy or benzhydryloxy; or a polymerizable group such as allyloxy or vinylphenylmethoxy. Such polymerizable groups can be polymerized, where necessary, after polymeric micelle comprising a coordination compound according to the present invention is formed, to make the structure of the polymeric micelle even more durable. The polymeric micelles formed of the coordination compounds of the present invention, however, are so considerably stable in aqueous media that the polymerization will be unnecessary.

Again referring to the formulae, m is an integer of 35 - 20,000, preferably 40 - 500; and n is an integer in the range of 15 to 30, preferably, 20 - 30, or around 20. In the copolymer, the degree of polymerization of P(Glu) chains or the average number of repeating units of Glu is not more than 30, but is at least 15, preferably at least 17, more preferably, at least 20. These numerical values signify the mean value (peak value) of molecular weight distribution, which can be determined when segment formation is completed. As stated also in Non-patent document 5, when a block copolymer of very narrow molecular weight distribution, for example, one having a Mm/Mn within a range of 1.04 - 1.1, a range comparable with Mm/Mn = 1.06, is used in the present invention and when its number of repeating units or degree of polymerization is selected as above, the intended effect of the present invention can be attained without fail. Those block copolymers having the narrow molecular weight distribution can be conveniently obtained in accordance with later-appearing Production Example.

Furthermore, approximately at least 80%, preferably about 100%, of the n R⁵s in the P(Glu) segment in a copolymer of the general formula (1-a) or (1-b) is hydrogen atom or an alkali metal ion. Where carboxyl-protective group(s) other than hydrogen atom or alkali metal ion are present, they can be present at random in the repeating units (Glu).

In the coordination compound of the present invention, the equivalent ratio (Pt/COO-) of the platinum (Pt) in 1,2-diaminocyclohexane platinum (II) moiety to the carboxyl group (carboxyl anion) in the block copolymer moiety derived from the block copolymer is preferably at least 0.05, more preferably at least 0.1, most preferably, 0.3 - 1.0, or 1.2. Theoretically, the upper limit of the equivalent ratio in the coordination compound could be 2.0. Whereas, when the coordination compound is used to provide an anti-cancer agent, it may contain DACHPt besides the diaminocyclohexane platinum (II) moiety constituting the coordination compound with the copolymer, within the limit in conformity with the objects of the present invention.

The coordination compounds according to the invention can be prepared by the method as described in Non-patent document 5. Specifically, it can be conveniently prepared by reacting DACHPt with the block copolymer in an aqueous solvent, at an equivalent ratio of the Pt to the carboxyl group and under the conditions as will enable bonding of platinum (Pt) in the DACHPt and carboxyl anions in the block copolymer moiety; and recovering the resulting coordination compound composed of the diaminocyclohexane platinum (II) moiety(ies) and the copolymer moiety(ies). Here the DACHPt may be reacted with silver nitrate in an aqueous solvent in advance to form diaquo DACH platin. After removing from the system silver chloride which is by-produced in that occasion, the remaining aqueous solution containing diaquo DACH platin can be subjected to the complex-producing reaction as it is.

While the reaction conditions are not limited and any conditions may be selected so long as the intended coordination compound can be obtained, it is recommendable to select the use ratio of the diaquo DACH platin and block copolymer such that the equivalent ratio of Pt to carboxyl group should become at least 0.1, preferably at least 0.3 and at the maximum 1; the concentration level at which the two reactants are dissolved or partially suspended; and the reaction temperature within a range of 5 - 60°C.

The term, aqueous medium or aqueous solvent, as used in the present specification signifies water (in particular, deionized water) which may contain various inorganic or organic buffers or water-miscible organic solvent such as acetonitrile, dimethylformamide, ethanol or the like, within a range as will not adversely affect the coordination compound-producing reaction. Thus produced coordination compound is normally present, when visually observed, as polymeric micelles which are solubilized or homogeneously suspended in the aqueous solvent (normally having an average particle size of 20 - 100 nm as measured with dynamic light-scattering method). Such polymeric micelles can be recovered from the reaction mixture by any of common methods for isolating and purifying particles from suspensions of nanocolloidal particles or nanoparticles (having nano-order average diameters). The typical of such methods include ultrafiltration, diafiltration and dialysis.Thus isolated and purified drug-encapsulating polymeric micellar solution can be directly sterilized and used as an injection, after addition of per se known auxiliaries or excipient suitable for injections, where necessary. The polymeric micellar solution may also be concentrated and, for example, lyophilized to provide a fine solid powder. The fine powder can be dissolved or dispersed again in injectable solutions at considerably high concentration. The drug-encapsulating polymeric micelles according to the present invention show low accumulation as compared with, for example, oxaliplatin, in the internal organs such as the kidney, the liver, the spleen and the like, in particular, the liver, and in consequence low systemic toxicity, Thus the micelles can be used with very high safety. They also exhibit significantly higher anti-cancer action as compared with that of oxaliplatin. When used as an injection, they can also form a medical preparation suitable for bolus administration.

The fine powder can be processed into preparation forms adapted for various administration routes, as blended with, where necessary, pharmaceutically acceptable excipients. Typical examples of such excipients include deionized water, aqueous solution buffered at a prescribed pH, oligo- or poly-ethylene glycol, monosaccharide or oligosaccharide, sugar alcohol and the like. It is preferable to provide it in a form of a composition suitable for parenteral administration, particularly intravenous or subcutaneous administration.

For example, in case of intravenous administration, the dosage should preferably be determined by a medical specialist, based on the results of small-scale experiments with laboratory animals or volunteers and also conditions of individual patients. Whereas, generally it can be 1.0 - 1000 mg/m² (surface area of the patient's generally it can be 1.0 - 1000 mg/m² (surface area of the patient's body) per administration per day, although not limited thereto. Suitable dosages can also be selected according to an administration schedule such as a few days' continuous administration of 10 - 200 mg/m² (surface area of the patient's body) once per day followed by a fixed period of non-administration, or administration of a dose of 50 - 500 mg/m² (surface area of the patient's body) once per day, followed by a few days' non-administration and re-administration.

The coordination compound of the present invention has anti-tumor activity spectrum similar to that of dichloro(1,2-diaminocyclohexane)platinum (II) or oxaliplatin. Hence the present invention also provides anti-cancer compositions containing the coordination compound as the active ingredient.

According to the invention, medical preparations or medical compositions for treating, for example, testicular tumor, bladder tumor, pelviureteral tumor, prostatic cancer, ovarian cancer, head and neck cancer, alveolar adenocarcinoma, esophageal carcinoma, endocervical carcinoma, neuroblastoma, stomach cancer and rectal cancer can be provided, although not limited thereto.

The coordination compound or the drug-encapsulating polymeric micelles according to the invention can be used in combination with other anti-tumor agents or anti-cancer agents. As these anti-cancer agents, for example, cytarabine, 5-FU, doxorubicin, taxol, carboplatin, cisplatin and the like can be named, while any of those agents can be used so long as its combined use does not produce an adverse effect. Whereas, combined use of the cisplatin-encapsulating polymeric micells as described in the earlier cited WO 02/26241A1 (or a coordination compound of the copolymer and cisplatin) with the coordination compound of the present invention is particularly preferred.

### Brief explanation of drawings

Fig. 1 shows GPC chart of the PEG-PBLG 12 - 20 which was obtained in Production Example 1.
Fig. 2 shows ¹H-NMR chart of PEG-P(Glu) 12 - 20 (measuring conditions: concentration 10 mg/mL, solvent: DMSO-d₆, integration number: 64, temperature: 25°C.)
Fig. 3 is a distribution diagram showing the result of dynamic light scattering (DLS) measurement of DACHPt-encapsulating polymeric micelles.

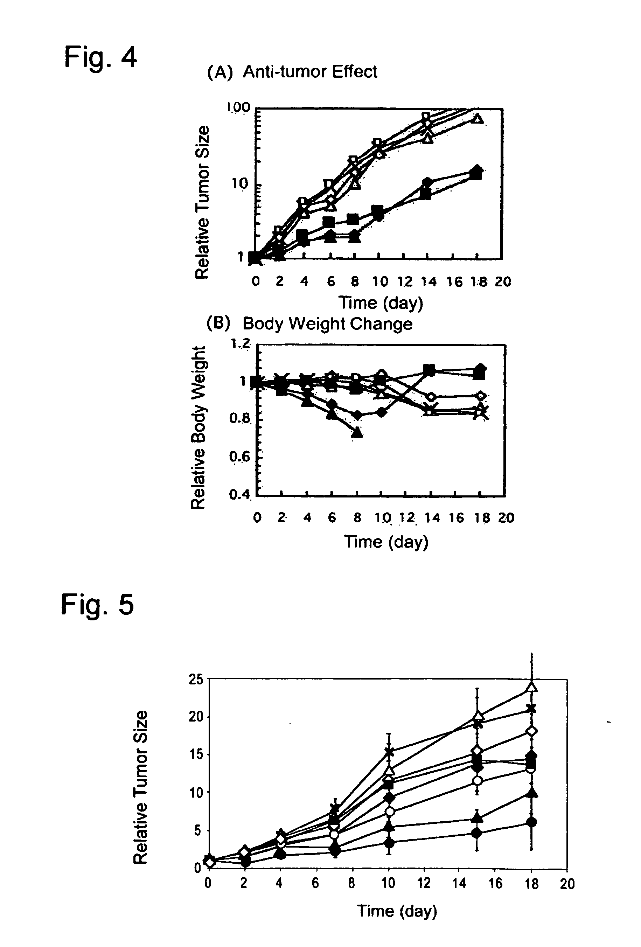

In Fig. 4, (A) is a graph showing anti-tumor effect of the drugs which were intravenously administered to experimental animals (the axis of ordinates represents relative tumor size and the axis of abscissae, time (day)); and (B) is a graph showing the body weight changes (the axis of ordinates represents relative body weight of the animals and the axis of abscissae, time (day)). In the graphs, x marks are for untreated group (control), and other marks are for the groups treated with, respectively, △: oxaliplatin, 6 mg/kg, ◇: the same, 4 mg/kg, □: the same, 2 mg/kg, ◆ comparison DACHPt-encapsulating polymeric micelle (using PEG-P(Glu) 12 - 46), 4 mg/kg, ■ the same, 2 mg/kg, ▲: the same, 6 mg/kg.

Fig. 5 is a graph showing anti-tumor effect of the drugs intravenously administered to experimental animals (ordinate axis: relative size of tumor, abscissa axis: time (day)). ■: untreated group (control), •: DACHPt-encapsulating polymeric micelle of the present invention (using PEG-P(Glu) 12 - 20) 6 mg/kg, ▲: the same, 4 mg/kg, ◆: the same, 2 mg/kg, ○: oxaliplatin, 10 mg/kg, △: the same, 6 mg/kg, ◇: the same, 4 mg/kg, x: the same, 2 mg/kg.

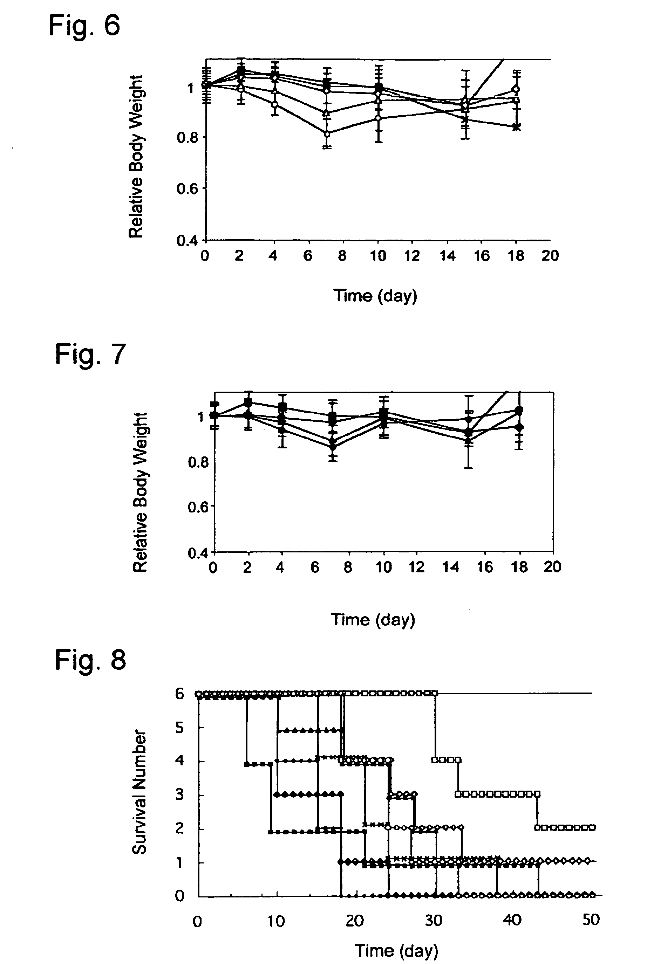

Fig. 6 is a graph showing body weight changes in experimental animals to which oxaliplatin was intravenously administered (ordinate axis: relative body weight of animal, abscissa axis: time (day)). ■: untreated group (control), ○: 10 mg/kg, △: 6 mg/kg, ◇: 4 mg/kg, x: 2 mg/kg.

Fig. 7 is a graph showing body weight changes in experimental animals to which DACHPt-encapsulating polymeric micelle (using PEG-P(Glu) 12 - 20) was intravenously administered (ordinaite axis: relative body weight of animal, abscissa axis; time (day)). ■: untreated group (control), •: 6 mg/kg, ▲: 4 mg/kg, ◆: 2 mg/kg.

Fig. 8 is a graph showing anti-tumor effect of the drugs which were intravenously administered to experimental animals (ordinate axis: surviving animal number, abscissa axis: survival time (day)). ◆: untreated group (control), ■: oxaliplatin, 10 mg/kg, ■: the same, 6 mg/kg, x: the same, 4 mg/kg, •: the same, 2 mg/kg, □: DACHPt-cncapsalating polymer micelle of the present invention (using PEG-P(Glu) 12 - 20), 6 mg/kg, ◇: the same, 4 mg/kg, ○: the same, 2 mg/kg.

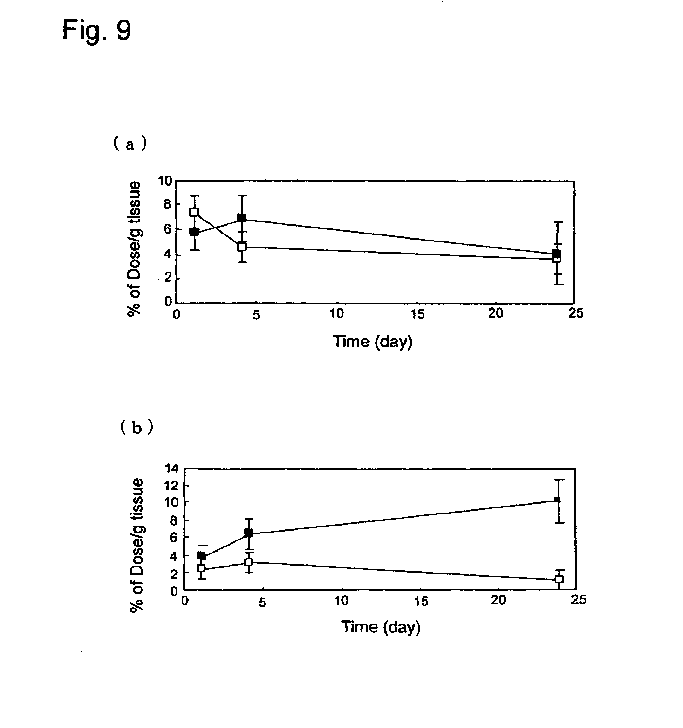

Fig. 9 are the graphs showing accumulation behavior of the drugs in the organs of experimental animals to which the drugs were intravenously administered (ordinate axis: platinum dosage % per weight of the organ, abscissa axis: time), (a) and (b) showing renal and hepatic accumulation, respectively. ■: comparison DACHPt-encapsulating polymer micelle (using PEG-P(Glu) 12 - 35), □: CDDP.

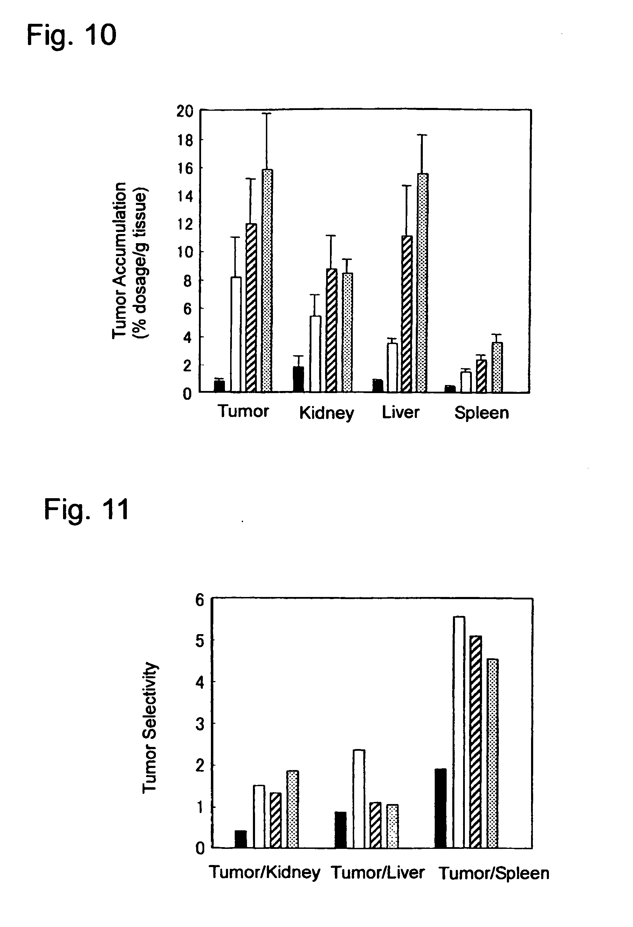

Fig. 10 is a graph showing accumulation of oxaliplatin and DACHPt-encapsulating micelles in tumor, kidney, liver and spleen of colon-26-transplanted CDF-1 mice, after 24 hours of intravenous administration of the drugs (in the graph, solid bars show the case of oxaliplatin administration; blank bars, the case of administering DACHPt-encapsulating PEG-P-(Glu) 12 - 20 micelles; the bars filled with diagonal lines, the case of administering DACHPt-encapsulating PEG-P(Glu) 12 - 40 micelles; and the bars filled with dots, the case of administering DACHPt-encapsulating PEG-P(Glu) 12 - 70 micelles).

Fig. 11 is a graph showing selective tumor accumulation of oxaliplatin and DACHPt-encapsulating micelles (in the graph, the bars have the same significations as explained as to Fig. 10).

### Best mode for practicing the invention

Hereinafter the present invention is explained still more specifically, referring to specific examples which, however, are not intended to limit the invention thereto.

### Production Example 1: Production of block copolymer

This is a production example of a block copolymer represented by the following structure formula:

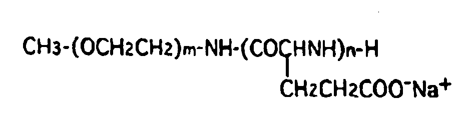

Using 1g of poly(ethylene glycol)(PEG) having a terminal primary amino group and a molecular weight of 12,000 (corresponding to about 273 of the average value of m) as the initiator, 0.43 g of γ-benzyl-L-glutamate was ring-opening-polymerized to provide PEG-poly (γ-benzyl-L-glutamic acid) [-(PBLG)]. Synthesis of the mono peak block copolymer was confirmed by gel permeation chromatography (GPC) [conditions: concentration 1 mg/ml, column; TSK-gel G4000HHR+G3000HHR, effluent; N,N'-dimethylformamide (DMF) + 10 mM LiCl, flow rate; 0.8 mL/min, detection; differential refratometer, temperature; 40°C] (Figure 1).

The PEG-P(Glu) used in the present invention was prepared by alkali hydrolyzing and deprotecting benzyl esters of PEG-PBLG with 0.5N NaOH. The composition of the block copolymer was confirmed by means of ¹H-NMR measurement of the product, to find that the degree of polymerization of the PBLG was 20 (Fig. 2).

In the following Examples, the block copolymers are identified as (molecular weight to PEG × 10⁻³) - (polymerization degree of polyamio acid): for example, PEG-P(Glu) 12 - 20 means that the average molecular weight of PEG is about 12000 and the average degree of polymerization of P(Glu) is about 20.

### Production Example 2: Production of the coordination compound

One-hundred (100) mg of dichloro(1,2-diaminocyclohexane)-platinum (II) (DACHPt) was suspended in water, to which one molar ratio thereto of silver nitrate was added to convert the DACHPt to a water-soluble hydrate complex having higher water-solubility. The silver chloride formed as a precipitate was removed by centrifugation and then filtration through a 0.22 µm filter. The polymeric micelles (DACHPt-encapsulating polymeric micelles) formed of the coordination complex composed of the (1,2-diaminocyclohexane) mixing the hydrate complex corresponding to the DACHPt and PEG-P(Glu) 12 - 20 at a molar ratio of DACH-Pt to the Glu residue of 1.0 in water at 37°C and reacting them for 120 hours. The resulting polymeric micelles were purified by ulrafiltration (cutoff molecular weight: 100,000). The result of their particle size measurement by dynamic light scattering (DLS) method was as shown in Fig. 3. From the Fig. 3, formation of mono-dispersed particles having an average particle diameter of about 40 nm was confirmed.

### Treating Test Example 1 (control):

To CDF1 mice (n=4, female, 6 weeks) with mouse colon cancer Colon-26 cell line transplanted at their lower abdominal region, oxaliplatin (control) and DACHPt-encapsulating polymeric micelle formed of PEG-P(Glu) 12 - 46 (derived from the block copolymer as described in Non-patent document 5: comparative example) each were administered via their caudal vein. The volume changes in the tumor in the mice and the body weight changes of the mice were as shown in Fig. 4(A) and (B), respectively. In this experiment, the tumor was allowed to grow for 9 days after the transplantation, and the therapeutic treatment was started after the tumor size reached about 100 mm³. Also in this experiment, the therapy was conducted by administering oxaliplatin or DACHPt-encapsulating polymeric micelles at the doses of each 2, 4 and 6 mg/kg in terms of DACHPt, on every third day, four times. The DACHPt- encapsulating polymeric micelles did not show any significant toxicity at the dose of 2 mg/kg and exhibited markedly higher anti-tumor effect compared with oxaliplatin, but at the doses of 4 and 6 mg/kg, exhibited toxicity after eighth day of the treatment (after the fourth administration) and the mice' body weight decreased to 80% or less. Particularly, at the dose of 6 mg/kg, all of the treated mice died. One reason for the deaths is considered to be non-specific accumulation of the DACHPt-encapsulating polymeric micelles formed of PEG-P(Glu) 12 - 46 in the liver (see later given Comparative Treating Test Example).

### Treating Test Example 2 (This invention)

Similarly to Treating Test Example 1 (control), to CDF1 mice (n=6, female, 6 weeks) with mouse colon cancer Colon-26 cell line transplanted at their lower abdominal region, oxaliplatin (control) and DACHPt-encapsulating polymeric micelles prepared as in Production Example 2 using PEG-P(Glu) 12 - 20 were administered via their caudal vein. The tumor volume changes in the treated mice were as shown in Fig. 5. Also the body weight changes of the mice treated with oxaliplatin and those of the mice treated with the DACHPt-encapsulating polymeric micelles formed from PEG-P(Glu) 12 - 20 in this experiment were as shown in Figs. 6 and 7, respectively. In this experiment, the treatment was started on the 9th day after the tumor transplantation. The treatment consisted of administration of prescribed doses of oxaliplatin (2, 4, 6 and 10 mg/kg) or DACHPt-encapsulating polymeric micelles (2, 4 and 6 mg/kg as converted to DACHPt) on every third day, three times. In comparison with the oxaliplatin-treated group (doses = 2 - 10 mg/kg), the DACHPt-encapsulating polymeric micelles exhibited remarkable anti-tumor effect at the doses of 4 and 6 mg/kg (Fig. 5). Nevertheless, the DACHPt-encapsulating polymeric micelles (4 and 6 mg/kg) did not show lethal systemic toxicity (Fig. 7).

On the other hand, the remarkable tumor growth- inhibiting effect as seen in the DACHPt-encapsulating polymeric micelle-administered group was not observed with oxaliplatin, even when the treatment was given at the doses which brought about the body weight reduction to 80% (10 mg/kg, Fig. 6). The survival number of the mice (individual number) in the experiment is shown in Fig. 8. The 10 mg/kg oxaliplatin-administered mice group showed earlier deaths than the untreated group (control), and presumably that dosage induces lethal toxicity. With the mice treated with oxaliplatin at lower doses (2, 4 or 6 mg/kg), little life-prolonging effect was recognized. By contrast, the DACHPt-encapsulating micelles formed from PEG-P(Glu) 12 - 20 showed distinct life-prolongation effect at all the dose levels tested (Fig. 8). In particular, the treatment with DACHPt-encapsulating polymeric micelles at the treatment with DACHPt-encapsulating polymeric micelles at the dose of 2 mg/kg caused no body weight decrease (Fig. 7) and very remarkable life-prolongation effect (by one month or more) was confirmed (Fig. 8). From the foregoing results, it is concluded that the DACHPt-encapsulating polymeric micelle formed from PEG-P(Glu) 12 - 20 is a markedly superior medical preparation to oxaliplatin, in both aspects of toxicity and therapeutic effect.

Those results signify that DACHPt-encapsulating polymeric micelle produced from PEG-P(Glu) 12 - 20 can exhibit higher therapeutic effect (life prolongation effect) and reduction in toxicity at high doses (i.e., it expands the effective therapeutic range ensuring therapeutic effect and safety), in comparison with the DACHPt-encapsulating polymeric micelle which was formed from PEG-P(Glu) 12 - 46 following Non-patent document 5 and which exhibited in the Treating Test Example 1 higher tumor growth-inhibitory effect than oxaliplatin, but toxicity at high doses, inducing appreciable body weight reduction at the doses not less than 2 mg/kg (Fig. 4). The toxicity of DACHPt-encapsulating polymeric micelles formed from PEG-P(Glu) 12 - 46 is considered to be induced by its notable accumulation in the liver. Whereas, the DACHPt-encapsulating polymeric micelles which are formed from PEG-P(Glu) 12 - 20 presumably have low accumulation in the liver and probably the toxicity is drastically reduced thereby. Namely, adequate selection of polyglutamic acid (P(Glu)) chain length in PEG-P(Glu) block copolymer is found important for inhibiting hepatic accumulation of DACHPt- encapsulating polymeric micelles to ensure high safety and therapeutic effect; and the presence of critical chain length is recogonized.

### Other comparative tests (referential examples)

### (a) In vitro cytotoxicity test

Cytotoxicity test of DACHPt-encapsulating polymeric micelles formed by the above-described procedures, against mouse Colon 26 (C-26) cell line was conducted.

Growth-inhibitory activity against mouse Colon 26 (C-26) cell line, of CDDP-encapsulating polymeric micelles prepared with PEG-P(Glu) 12 - 35 (see: Patent document 4) and the above-described DACHPt-encapsulating polymeric micelles was evaluated by MTT method. Five-thousand (5,000) cells were incubated in a 96 well-multiplate supplied with 50 µl of RPMI 1640 medium containing 10% fetal bovine serum. The cells were exposed to the drugs for 48 and 72 hours. MTT solution was added, and 150 µl of a 0.01 M HCl-added solution containing 10 wt% of sodium dodecylsulfate (SDS) was added 6 hours later. Viability of the cells was then measured based on optical density at 570 nm of formazan produced 24 hours later. From the measured results, 50% inhibition concentration (IC₅₀) values at 48 hours' incubation and 72 hours' incubation were determined, which were 2.3 µg/m and 1.5 µg/ml for DACHPt-encapsulatinog polymeric micelles, respectively, and 20.3 µg/m and 7.5 µg/m for CDDP-encapsulating polymeric micelles, respectively.

### (b) In vivo distribution

Balb/c mice (n=3, female, 6 weeks, 20 to 25 g) were subcutaneously inoculated with C-26 cell line (1×10⁶) at right side of the abdomen. Free CDDP and DACHPt-encapsulating polymeric micelle (100 µg each, in terms of the respective drug) were administered through the mice' caudal vein, on 21st day from the tumor inoculation. After 1, 4 and 24 hours after the administration, from the mice the livers and kidneys were excised. The organs were weighed and dissolved in warm nitric acid. The solutions were dried. The amount of platinum was measured by ICP-MS immediately after addition of hydrochloric acid. The result is shown in Fig. 9 (a) and (b), (a) showing renal accumulation behavior of each drug, and (b) showing hepatic accumulation behavior of each drug. From the results it can be understood that DACHPt micelles remain in blood flow for a long period and accumulates effectively in solid cancer without specific accumulation in normal tissues. DACHPt polymeric micelles prepared from PEG-P(Glu) 12 - 35 achieved significantly higher inhibition of cell proliferation against the tested tumor cell strain, than that of the corresponding CDDP-encapsulating polymeric micelles, but showed the tendency of acculating in the liver with time.

The DACHPt encapsulating polymeric micelle used in the above experiment was produced as follows: 19 mg of DACHPt was suspended in 10 ml of distilled water, and the suspension was mixed with 12.3 mg of silver nitrate (molar ratio of [AgNO₃]/[DACHPt] = 1.5). The liquid mixture was kept in a dark place at 25°C for 24 hours. After the reaction, silver chloride precipitated. Then, the reaction mixture was centrifuged at 3,000 rpm for 5 minutes to remove the precipitate of silver chloride. Thus obtained diaquo DACHPt-containing supernatant was purified by filtration through a 0.22 mm filter. Separately, 24.4 mg of a block copolymer of the structural formula as given in Production Example 1, whose m value was such that the PEG segment in PEG-P(Glu) had a number-average molecular weight of about 12,000 and the average value of n P(Glu) was about 35 (this copolymer is hereafter referred to as PEG-P(Glu) 12 - 35) was prepared and added to 10 ml of the above supernatant (the molar ratio of [diaquo-DACHPt]/[Glu] = 1). Thus obtained solution was processed as in Production Example 2 to provide the DACHPt-encapsulating polymeric micelle.

### Experiment 3: Evaluation of in vivo distribution of DACHPt-encapsulating polymeric micelles prepared from block copolymers of different construction

CDF-1 mice (n = 3, female, six weeks, 20 - 25 g) were subcutaneously inoculated with C-26 cell line (1 × 10⁶) at right side of the abdomen. On the 14th day after the tumor inoculation, free oxaliplatin or DACHPt-encapsulating polymeric micelles which were formed from PEG-P(Glu) 12 - 20, 12 - 40 and 12 - 70, respectively (each at dose of 100 µg in terms of the respective drug) were administered to the mice through the caudal vein. On the 24th hour after the administration, the tumor, kidneys, livers and kidneys were excised from the mice. The organs were weighed, and then they were dissolved in warm nitric acid. The solutions were dried. Their platinum contents were measured by ICP-MS immediately after the addition of hydrochloric acid. The result is shown in Fig. 10(A). Those DACHPt-encapsulating polymeric micelles prepared from PEG-P(Glu) 12 - 20, 12 - 40 and 12 - 70 showed 11, 16 and 21 times solid cancer accumulation, respectively, that of oxaliplatin. On the other hand, the DACHPt-encapsulating polymeric micelles prepared from PEG-P-(Glu) 12 - 20, 12 - 40 and 12 - 70 showed 4.4, 14 and 20 times accumulation in the liver which was normal, respectively, that of oxaliplatin. Thus, clearly the polymeric micelle formed of PEG-P(Glu) 12 - 20 has low hepatic accumulation. Similarly, it was confirmed that the polymeric micelle formed from PEG-P(Glu) 12 - 20 has lower accumulation tendency in the kidney and spleen, compared with the polymeric micelles of different constructions.

For the purpose of evaluating selectivity for solid cancer accumulation, quotients of the tumor accumulation divided by the normal organ accumulation are shown in Fig. 10(B). The results show that all of the DACHPt-encapsulating polymeric micelles exhibited higher tumor selectivity than oxaliplatin, irrelevantly to their construction. In particular, it is made clear that the polymeric micelle formed from PEG-P(Glu) 12 - 20 has higher tumor selectivity compared with other drugs.

As is clear from the foregoing, accumulation of DACHPt-encapsulating polymeric micelles in normal tissues (in particular, the liver) depends on the construction of PEG-P(Glu), and the critical chain length of its P(Glu) is assumed to be present between 20 and 40. That is, at the chain lengths not longer than the critical chain length, DACHPt-encapsulating polymeric micelles accumulate in solid concer while showing low accumulation in normal tissues, and achieve high tumor selective accumulation.

## Claims

1. A coordination compound comprising (1,2-diaminocyclohexane)-paltinum(II) moiety and block copolymer moiety derived from a block copolymer of the general formula (1-a) or (1-b): 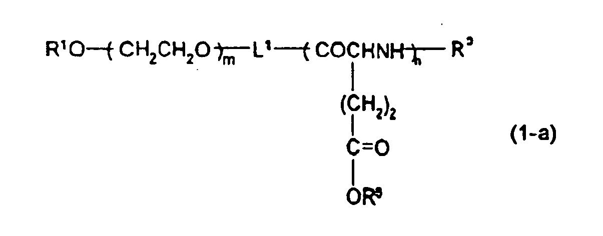 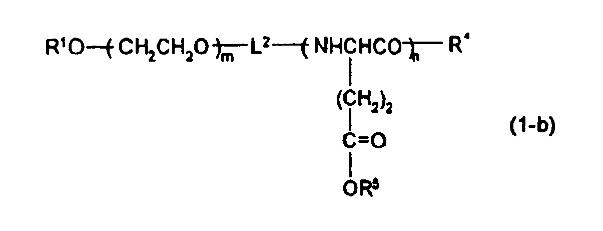 (in the formulae, R¹ stands for hydrogen atom or an unsubstituted or substituted, straight chain or branched chain C₁₋₁₂ alkyl; L¹ and L² each stands for a linking group; R³ stands for hydrogen atom, an amino-protective group, hydrophobic group or polymerizable group; R⁴ stands for hydroxyl or an initiator residue; R⁵ each independently stands for hydrogen atom, an alkali metal ion or carboxyl-protective group; m is an integer of 35 - 20,000; and n is an integer in the range of 15 to 30, with the proviso that hydrogen atom or an alkali metal ion occupies at least 80% of n R⁵s) the coordination compound being formed through a coordination bond between the platinum in the (1,2-diaminocyclohexane)platinum(II) moiety and one or two carboxylate groups in the block copolymer moiety.

2. The coordination compound as set forth in Claim 1, in which all of the R⁵s in the block copolymer represented by the general formula (1-a) or (1-b) are hydrogen atoms or alkali metal ions.

3. The coordination compound as set forth in Claim 1, in which, where the coordination bond is formed through the platinum in the (1,2-diaminocyclohexane)platinum(II) moiety and one carboxylate group in the block copolymer moiety, aqua or chloro ligand(s) bind to the remaining bonding hand(s) of the platinum.

4. The coordination compound as set forth in Claim 1, in which L¹ is -(CH₂)_{b}-NH-, b being an integer of 1 - 5 and L² is - (CH₂)_{c}-CO-, c being an integer of 1 - 5.

5. The coordination compound as set forth in Claim 1, in which m is an integer of 40 - 500, and n is 17 - 30.

6. The coordination compound as set forth in Claim 1 which is capable of forming, in an aqueous medium, a polymeric micelle in such a form that nanoparticles with the poly(ethylene glycol) segments in the block copolymer of the general formula (1-a) or (1-b) serving as the shell and the poly(glutamic acid) segments serving as the core, encapsulate 1,2-diaminocyclohexane platinum(II).

7. The coordination compound as set forth in Claim 1, in which the equivalent ratio (Pt/COO-) of platinum (Pt) in the (1,2-diaminocyclohexane)- platinum (II) moiety to carboxyl group in the block copolymer moiety is 0.3 - 1.

8. An anti-cancer composition comprising the coordination compound as set forth in Claim 1 and pharmaceutically acceptable excipients.

9. Use of the coordination compound as set forth in Claim 1, for formulating medical preparations for treating cancer patients.

10. A coordination compound as set forth in Claim 1 for use in a method for treating cancer patients.

## Patentansprüche

1. Koordinationsverbindung, umfassend eine (1,2-Diaminocyclohexan)platin(II)-Baugruppe und eine Blockcopolymerbaugruppe, abgeleitet von einem Blockcopolymer der allgemeinen Formel (1-a) oder (1-b): 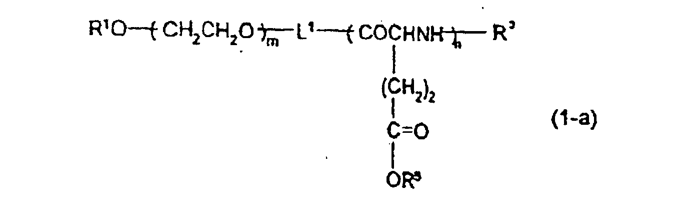 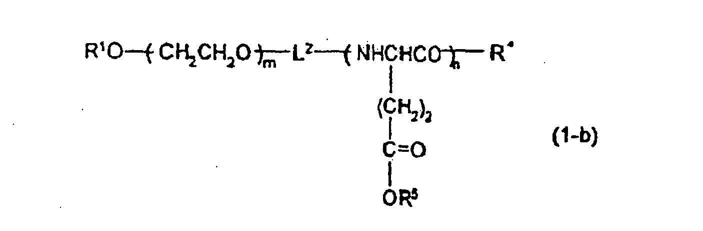 (in den Formeln steht R¹ für ein Wasserstoffatom oder unsubstituiertes oder substituiertes, geradkettiges oder verzweigtes C₁₋₁₂-Alkyl; L¹ und L² stehen jeweils für eine Verknüpfungsgruppe; R³ steht für ein Wasserstoffatom, eine Aminoschutzgruppe, eine hydrophobe Gruppe oder eine polymerisierbare Gruppe; R⁴ steht für Hydroxyl oder einen Starterrest; R⁵ steht jeweils unabhängig für ein Wasserstoffatom, ein Alkalimetallion oder eine Carboxylschutzgruppe; m ist eine ganze Zahl von 35-20000; und n ist eine ganze Zahl im Bereich von 15 bis 30, mit der Maßgabe, dass das Wasserstoffatom oder Alkalimetallion mindestens 80% der n R⁵ besetzt) wobei die Koordinationsverbindung gebildet wird durch eine Koordinationsbindung zwischen dem Platin in der (1,2-Diaminocyclohexan)platin(II)-Baugruppe und einer oder zwei Carboxylatgruppen in der Blockcopolymerbaugruppe.

2. Koordinationsverbindung gemäß Anspruch 1, in welcher alle der R⁵ im Blockcopolymer, dargestellt durch die allgemeine Formel (1-a) oder (1-b), Wasserstoffatome oder Alkalimetallionen sind.

3. Koordinationsverbindung gemäß Anspruch 1, in welcher, wosofern die Koordinationsbindung durch das Platin in der (1,2-Diaminocyclohexan)platin(II)-Baugruppe und einer Carboxylatgruppe in der Blockcopolymerbaugruppe gebildet wird, eine oder mehrere Wasser- oder Chlorligand(en) an die verbleibende(n) Bindungsseite(n) des Platins binden.

4. Koordinationsverbindung gemäß Anspruch 1, wobei L¹ -(CH₂)_{b}-NH- ist, wobei b eine ganze Zahl von 1-5 ist, und L² -(CH₂)_{c}-CO- ist, wobei c eine ganze Zahl von 1-5 ist.

5. Koordinationsverbindung gemäß Anspruch 1, wobei m eine ganze Zahl von 40-500 ist und n 17-30 ist.

6. Koordinationsverbindung gemäß Anspruch 1, welche in der Lage ist, in einem wässrigen Medium eine polymere Mizelle in einer solchen Form zu bilden, dass Nanopartikel mit den Poly(ethylenglykol)-Segmenten im Blockcopolymer der allgemeinen Formel (1-a) oder (1-b), die als die Hülle dienen, und den Poly(glutaminsäure)-Segmenten, die als der Kern dienen, 1,2-Diaminocyclohexanplatin(II) einkapseln.

7. Koordinationsverbindung gemäß Anspruch 1, wobei das Äquivalenzverhältnis (Pt/COO-) von Platin (Pt) in der (1,2-Diaminocyclohexan)platin(II)-Baugruppe zur Carboxylgruppe in der Blockcopolymerbaugruppe 0,3-1 beträgt.

8. Anti-Krebs-Zusammensetzung, umfassend die Koordinationsverbindung gemäß Anspruch 1 und pharmazeutisch akzeptable Exzipienzien.

9. Verwendung der wie in Anspruch 1 beschriebenen Koordinationsverbindung zur Formulierung medizinischer Zubereitungen zur Behandlung von Krebspatienten.

10. Koordinationsverbindung gemäß Anspruch 1 zur Verwendung in einem Verfahren zur Behandlung von Krebspatienten.

## Revendications

1. Composé de coordination comprenant le fragment (1,2-diaminocyclohexane) platine (II) et le fragment de copolymère séquencé dérivé d'un copolymère séquencé de formule générale (1-a) ou (1-b) : 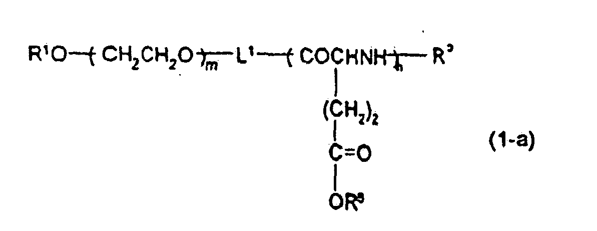 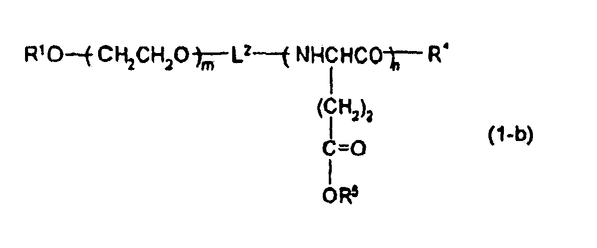 (dans les formules, R¹ désigne un atome d'hydrogène ou un C₁₋₁₂ alkyle à chaîne linéaire ou ramifiée substituée ou non substituée ; L¹ et L² désignent chacun un groupe de liaison ; R³ désigne un atome d'hydrogène, un groupe amino protecteur ou un groupe polymérisable ; R⁴ désigne un hydroxyle ou un résidu initiateur ; R⁵ désigne chacun indépendamment un atome d'hydrogène, un ion de métal alcalin ou un groupe carboxyle protecteur ; m est un entier de 35 à 20 000 ; et n est un entier dans le domaine de 15 à 30, à la condition qu'un atome d'hydrogène ou un ion de métal alcalin occupe au moins 80 % de n R⁵) le composé de coordination étant formé grâce à une liaison de coordination entre le platine dans le fragment (1,2-diaminocyclohexane) platine (II) et un ou deux groupes carboxylates dans le fragment de copolymère séquencé.

2. Composé de coordination selon la revendication 1, dans lequel l'ensemble des R⁵ dans le copolymère séquencé représenté par la formule générale (1-a) ou (1-b) sont des atomes d'hydrogène ou des ions de métal alcalin.

3. Composé de coordination selon la revendication 1, dans lequel, quand la liaison de coordination est formée grâce au platine dans le fragment (1,2-diaminocyclohexane) platine (II) et un groupe carboxylate dans le fragment de copolymère séquencé, un ou des ligand(s) aqua ou chloro se lie(nt) au(x) bras de liaison restant(s) du platine.

4. Composé de coordination selon la revendication 1, dans lequel L¹ est -(CH₂)-NH-, b étant un entier de 1 à 5 et L² est -(CH₂)_{c}-CO-, c étant un entier de 1 à 5.

5. Composé de coordination selon la revendication 1, dans lequel m est un entier de 40 à 500, et n est égal à 17 à 30.

6. Composé de coordination selon la revendication 1 qui est capable de former, dans un milieu aqueux, une micelle polymérique sous une forme telle que des nanoparticules avec les segments de poly(éthylène glycol) dans le copolymère séquencé de formule générale (1-a) ou (1-b) servant d'enveloppe et les segments de poly (acide glutamique) servant de noyau, encapsulent le (1,2-diaminocyclohexane) platine (II).

7. Composé de coordination selon la revendication 1, dans lequel le rapport équivalent (Pt/COO-) de platine (Pt) dans le fragment (1,2-diaminocyclohexane) platine (II) par rapport au groupe carboxyle dans le fragment de copolymère séquencé est de 0,3 à 1.

8. Composition anticancéreuse comprenant le composé de coordination selon la revendication 1 et des excipients pharmaceutiquement acceptables.

9. Utilisation du composé de coordination selon la revendication 1, pour formuler des préparations médicales pour traiter des patients cancéreux.

10. Composé de coordination selon la revendication 1 destiné à une utilisation dans un procédé pour traiter des patients cancéreux.
